# FASCICULE DE BREVET EUROPEEN

(11) **EP 3 755 200 B1**
(45) Date de publication et mention de la délivrance du brevet: **30.03.2022**
(21) Numéro de dépôt: 19706279.7
(22) Date de dépôt: 21.02.2019
(51) Int. Cl.: A61B 3/00, A61B 3/032, A61B 3/036

(54) **PROCÉDÉ DE TEST VISUEL, MODULE DE COMMANDE ET SYSTEM ASSOCIÉS**
SEHTESTVERFAHREN SOWIE ZUGEHÖRIGES STEUERMODUL UND SYSTEM
EYESIGHT TESTING METHOD, AND ASSOCIATED CONTROL MODULE AND SYSTEM

(30) Priorité: 23.02.2018 FR 1851618
(43) Date de publication de la demande: 30.12.2020
(73) Titulaire: SIVIEW, 91460 Marcoussis (FR)
(72) Inventeur: GUILLEMAIN, Nathalie, 95320 Saint Leu la Forêt (FR); PICHEREAU, Laure, 91460 Marcoussis (FR)
(74) Mandataire: Cabinet Camus Lebkiri
(86) Numéro de dépôt international: PCT/EP2019/054374
(87) Numéro de publication internationale: WO 2019/162412

(56) Documents cités:
- EP-A1- 1 844 701
- WO-A1-02/00105
- WO-A2-2011/135364
- WO-A2-2017/070704
- US-A1- 2012 212 706

## Description

### DOMAINE TECHNIQUE DE L'INVENTION

Le domaine de l'invention est celui des examens de vue qui, pratiqués sur un sujet, permettent de déterminer s'il a besoin d'une correction et laquelle.

L'invention se rapporte plus particulièrement à un procédé de test visuel par exemple lors d'un examen de vue, au moyen d'un support qui est soit une tête de réfracteur, automatique ou manuelle, soit une paire de lunettes d'essai, et permettant d'objectiver une mesure d'acuité visuelle. L'invention concerne également un module de commande pour la mise en œuvre du procédé de test visuel ainsi qu'un système comportant le module de commande, un écran de sélection et un écran de lecture pour la mise en œuvre du procédé de test visuel.

### ETAT DE LA TECHNIQUE ANTERIEURE

Un examen de vue d'un sujet débute par une mesure de réfraction objective et se poursuit par une mesure de réfraction subjective.

La mesure de réfraction objective est généralement réalisée au moyen d'un autoréfractomètre. Alternativement à l'utilisation d'un autoréfractomètre, une technique manuelle de skiascopie est parfois utilisée. A défaut l'opérateur peut partir de la précédente correction du sujet examiné.

Lors de la mesure de réfraction subjective, l'opérateur fait tester différentes corrections visuelles au sujet examiné au moyen d'un matériel adapté qui peut être :
- une paire de lunettes d'essai avec des verres d'essai,
- une tête de réfracteur manuelle ou
- une tête de réfracteur automatique.
Dans le premier cas, l'opérateur change manuellement les verres d'essai de la paire de lunettes d'essai. Avec une tête de réfracteur, l'opérateur fait défiler différents verres devant les yeux du sujet examiné, au moyen de mollettes dans le cas d'une tête de réfracteur manuelle ou via une console de commande dans le cas d'une tête de réfracteur automatique.

Au cours d'une telle mesure de réfraction subjective, l'opérateur mesure successivement l'acuité visuelle du sujet examiné en fonction de différentes corrections visuelles testées. Plus généralement, mesurer l'acuité visuelle du sujet examiné portant une correction visuelle donnée est une étape de nombreux tests visuels.

L'opérateur agence un verre optique devant un œil du sujet examiné (cas monoculaire) ou devant les deux yeux du sujet examiné (cas binoculaire) pendant que ce dernier observe un écran de lecture sur lequel un ou plusieurs symboles de test sont affichés. Le ou les symboles de test sont généralement des lettres ou des dessins. L'opérateur demande au sujet examiné de reconnaître le ou les symboles de test. L'opérateur valide ou non le test en cours en fonction des réponses données par le sujet examiné. Une mesure d'acuité visuelle est réalisée en monoculaire (un seul des deux yeux testé) ou en binoculaire (les deux yeux simultanément testés). En binoculaire, la correction testée peut être la même pour les deux yeux, ou bien différente pour chacun des deux yeux.

La manière dont l'opérateur apprécie et interprète les réponses données par le sujet examiné afin de valider ou non le test en cours est subjective, elle dépend de l'opérateur et deux opérateurs différents risquent de ne pas traiter de la même manière des réponses pourtant identiques. Un opérateur peut être tenté de valider une réponse fausse mais proche de la bonne réponse, par exemple « O » à la place de « D » ou « Q », etc. Cela a pour conséquence que la mesure d'acuité visuelle réalisée est peu fiable. Le document US 2012/212706 divulgue un procédé de test visuel selon le préambule de la revendication 1 et un système selon le préambule de la revendication 7.

Il est donc recherché une solution permettant, dans le cadre d'un examen de vue ou d'une vérification de correction visuelle portée, de mesurer une acuité visuelle de manière plus objective que dans l'art antérieur.

### EXPOSE DE L'INVENTION

Dans ce contexte, l'invention vise à remédier à tout ou partie des inconvénients de l'état de la technique identifiés ci-dessus.

A cette fin, un premier aspect de l'invention concerne un procédé de test visuel comportant une étape d'agencement d'un verre optique dans un emplacement d'un support et les étapes suivantes selon lesquelles un module de commande :
- affiche un symbole de test ou une pluralité de symboles de test sur un écran de lecture et sur un écran de sélection et affiche en outre un symbole de validation au moins sur l'écran de sélection ;
- reçoit une sélection d'un symbole de test ou du symbole de validation sur l'écran de sélection ;
le procédé étant tel que :
- lorsque le module de commande reçoit une sélection d'un symbole de test, le module de commande sélectionne ledit symbole de test en modifiant au moins une caractéristique d'aspect dudit symbole de test de manière qu'il passe d'un aspect initial à un aspect modifié qui le différencie des symboles de test non sélectionnés ;
- lorsqu'un premier temps prédéterminé s'est écoulé depuis la dernière sélection d'un symbole de test ou depuis l'affichage du symbole de test ou de la pluralité de symboles de test, le module de commande modifie au moins une caractéristique d'aspect du symbole de validation ;
- le module de commande met fin au procédé de test visuel dès qu'un nombre prédéterminé de symboles de test a été sélectionné.

Un deuxième aspect de l'invention concerne un module de commande pour la mise en œuvre du procédé de test visuel selon le premier aspect de l'invention, le module de commande ayant :
- des moyens pour afficher un symbole de test ou une pluralité de symboles de test sur un écran de lecture et sur un écran de sélection ;
- des moyens pour afficher un symbole de validation au moins sur l'écran de sélection ;
- des moyens pour recevoir une sélection d'un symbole de test ou du symbole de validation sur l'écran de sélection ;
les moyens d'affichage sur l'écran de sélection et l'écran de lecture et de réception d'une sélection sur l'écran de sélection étant tels que :
- lorsque le module de commande reçoit une sélection d'un symbole de test, le module de commande sélectionne ledit symbole de test en modifiant au moins une caractéristique d'aspect dudit symbole de test de manière qu'il passe d'un aspect initial à un aspect modifié qui le différencie des symboles de test non sélectionnés ;
- le module de commande modifie au moins une caractéristique d'aspect du symbole de validation, de manière qu'il passe d'un aspect initial à un aspect modifié, lorsque le premier temps prédéterminé s'est écoulé depuis la dernière sélection d'un symbole de test ou depuis l'affichage du symbole de test ou de la pluralité de symboles de test ;
- le module de commande met fin au procédé de test visuel dès qu'un nombre prédéterminé de symboles de test a été sélectionné.

Un troisième aspect de l'invention concerne un système pour la mise en œuvre d'un procédé de test visuel selon le premier aspect de l'invention, comportant un module de commande selon le deuxième aspect de l'invention ainsi qu'un écran de sélection et un écran de lecture.

Outre les caractéristiques qui viennent d'être mentionnées dans les paragraphes précédents, le procédé de test visuel selon le premier aspect de l'invention, le module de commande selon le deuxième aspect de l'invention et le système selon le troisième aspect de l'invention peuvent présenter une ou plusieurs caractéristiques complémentaires parmi les suivantes, considérées individuellement ou selon toutes les combinaisons techniquement possibles :
- Lorsque le premier temps prédéterminé s'est écoulé depuis l'affichage du symbole de test ou de la pluralité de symboles de test, indépendamment de la sélection ou absence de sélection d'un ou plusieurs symboles de test, le module de commande modifie au moins une caractéristique d'aspect du symbole de validation.
- Lorsque le module de commande reçoit une sélection d'un symbole de test qui est déjà sélectionné, le module de commande désélectionne ledit symbole de test en lui redonnant son aspect initial.
- Le module de commande met automatiquement fin au procédé de test visuel lorsqu'un deuxième temps prédéterminé s'est écoulé depuis la modification d'au moins une caractéristique d'aspect du symbole de validation par le module de commande.
- Le symbole de validation est un premier symbole de validation tant qu'aucun symbole de test n'a été sélectionné, ou un deuxième symbole de validation, distinct du premier symbole de validation, dès qu'au moins un symbole de test est sélectionné.
- Chaque symbole de test est :
   ∘ une lettre, ou
   ∘ un chiffre, ou
   ∘ un dessin, ou
   ∘ un E de Snellen, ou
   ∘ un C de Landolt.
- L'écran de sélection et l'écran de lecture sont deux écrans distincts.
- Alternativement, l'écran de sélection et l'écran de lecture sont un seul et même écran.

### BREVE DESCRIPTION DES FIGURES

D'autres caractéristiques et avantages de l'invention ressortiront à la lecture de la description qui suit, en référence aux figures annexées.
- La figure 1a montre un diagramme des étapes d'une première alternative du procédé de test visuel selon le premier aspect de l'invention.
- La figure 1b montre un diagramme des étapes d'une deuxième alternative du procédé de test visuel selon le premier aspect de l'invention.
- La figure 2 montre un agencement d'un verre optique dans un emplacement d'un support, selon une première étape du procédé de test visuel selon le premier aspect de l'invention.
- La figure 3a montre un premier exemple d'affichage, seulement sur un écran de sélection ou à la fois sur un écran de sélection et un écran de lecture, d'une pluralité de symboles de test et d'un symbole de validation.
- La figure 3b montre un deuxième exemple d'affichage, seulement sur un écran de sélection ou à la fois sur un écran de sélection et un écran de lecture, d'une pluralité de symboles de test et d'un symbole de validation.
- La figure 3c montre un troisième exemple d'affichage, seulement sur un écran de sélection ou à la fois sur un écran de sélection et un écran de lecture, d'une pluralité de symboles de test et d'un symbole de validation.
- La figure 3d montre un quatrième exemple d'affichage, seulement sur un écran de sélection ou à la fois sur un écran de sélection et un écran de lecture, d'une pluralité de symboles de test et d'un symbole de validation.

Pour plus de clarté, des éléments identiques ou similaires sont repérés par des signes de référence identiques sur l'ensemble des figures.

### DESCRIPTION DETAILLEE D'AU MOINS UN MODE DE REALISATION DE L'INVENTION

Un premier aspect de l'invention concerne un procédé de test visuel au moyen d'un module de commande, permettant d'objectiver une mesure d'acuité visuelle. Un deuxième aspect de l'invention concerne le module de commande pour la mise en œuvre du procédé de test visuel selon le premier aspect de l'invention. Un troisième aspect de l'invention concerne un système comportant le module de commande selon le deuxième aspect de l'invention, un écran de sélection et un écran de lecture, pour la mise en œuvre du procédé de test visuel selon le premier aspect de l'invention.

Dans le cadre de la présente invention, on entend par « verre optique » un élément optique de qualité adaptée à la réalisation de lunettes de vue, sans préjuger du matériau dans lequel il est réalisé et qui peut notamment être du verre ou du plastique. Les termes « verre ophtalmique » ou « verre correcteur » peuvent également être employés. Le terme « lentille optique » peut également être employé en toute rigueur, à condition de bien l'interpréter au sens d'un élément optique de qualité adaptée à la réalisation de lunettes de vue, par opposition à une lentille cornéenne ou lentille de contact.

### Procédé selon le premier aspect de l'invention

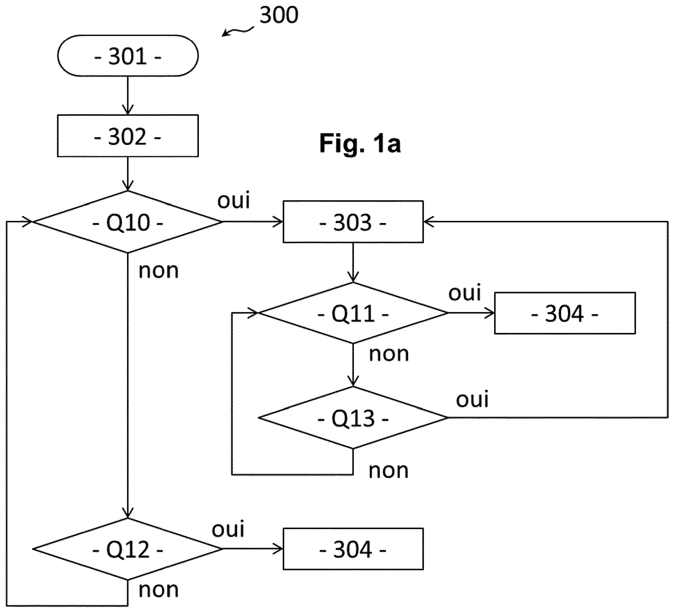

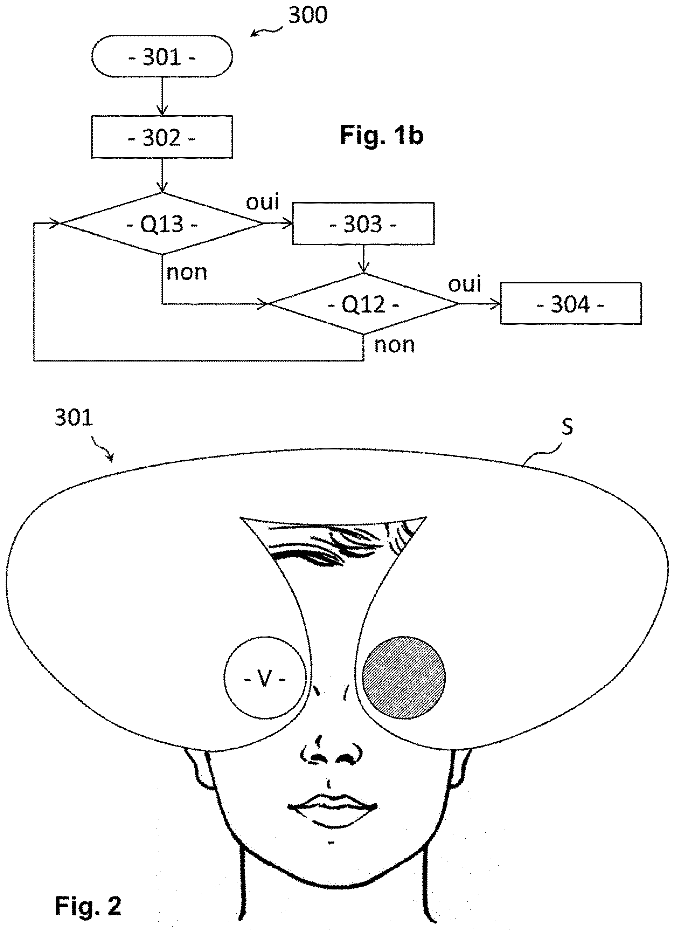

La figure 1a montre un diagramme des étapes du procédé 300 selon une première alternative. La figure 1b montre un diagramme des étapes du procédé 300 selon une deuxième alternative.

Dans la suite, on entend par « un opérateur sélectionne un symbole » le fait que l'opérateur réalise une action de sélection dudit symbole, en interaction avec l'écran de sélection. On entend par « le module de commande sélectionne un symbole » le fait que le module de commande valide la sélection reçue de l'opérateur et modifie au moins une caractéristique d'aspect dudit symbole de manière qu'il passe d'un aspect initial à un aspect modifié qui le différencie des symboles non sélectionnés. On entend par « le module de commande désélectionne un symbole de test » le fait que le module de commande modifie ledit symbole de test de manière qu'il perd son aspect modifié et reprend son aspect initial. On entend par « symbole de test sélectionné » un symbole de test sélectionné à la fois par l'opérateur et par le module de commande, c'est-à-dire un symbole de test pour lequel l'action de sélection de l'opérateur est validée par le module de commande. On entend par « symbole de test non sélectionné » un symbole de test non sélectionné par l'opérateur (pas d'action de sélection), ou un symbole de test sélectionné par l'opérateur et désélectionné par le module de commande c'est-à-dire pour lequel l'opération de sélection de l'opérateur est interprétée comme une désélection par le module de commande.

La première alternative de la figure 1a est à présent décrite. Selon une première étape 301 illustrée à la figure 2, un verre optique V est agencé dans un emplacement d'un support S qui est soit une tête de réfracteur, automatique ou manuelle, soit une paire de lunettes d'essai. Une tête de réfracteur ou une paire de lunettes d'essai sont tous les deux un support comportant des premier et second emplacements, un pour chaque œil, pouvant chacun recevoir un élément qui est soit un cache opaque, soit un verre optique. Dans le cas d'une paire de lunettes d'essai, l'opérateur manipule et agence manuellement les différents éléments dans les emplacements du support. Dans le cas d'une tête de réfracteur, l'opérateur ne manipule pas directement les différents éléments : il les agence dans les différents éléments du support en actionnant des mollettes dans le cas d'une tête de réfracteur manuelle, ou via une console de commande dans le cas d'une tête de réfracteur automatique. Le support est préférentiellement une tête de réfracteur automatique et dans ce cas, c'est le module de commande selon le deuxième aspect de l'invention qui assure la fonction de « console de commande » de la tête de réfracteur automatique.

Dans le cadre de la présente invention, on entend par « verre optique agencé dans un emplacement du support » soit un unique verre optique, soit une combinaison de plusieurs verres optiques, permettant de réaliser une certaine correction optique. Le support est destiné à être placé devant le sujet examiné de manière que le sujet examiné regarde, à travers les premier et second emplacements, un écran de lecture sur lequel au moins un symbole de test est affiché. Une étape d'agencement d'un élément dans un emplacement peut commencer le cas échéant par une étape de retrait d'un précédent élément dudit emplacement.

Dans le cadre du procédé de test visuel selon le premier aspect de l'invention, le support est utilisé en monoculaire œil droit ou en monoculaire œil gauche ou en binoculaire. En monoculaire œil droit, un verre optique est agencé dans l'emplacement correspondant à l'œil droit tandis qu'un cache est agencé dans l'emplacement correspondant à l'œil gauche. En monoculaire œil gauche, un verre optique est agencé dans l'emplacement correspondant à l'œil gauche tandis qu'un cache est agencé dans l'emplacement correspondant à l'œil droit. En binoculaire, un verre optique est agencé dans chacun des deux emplacements ; il peut s'agit d'un même verre optique, c'est-à-dire d'une même correction optique, dans chacun des deux emplacements, ou bien de deux verres optiques différents, c'est-à-dire de deux corrections optiques différentes dans chacun des deux emplacements.

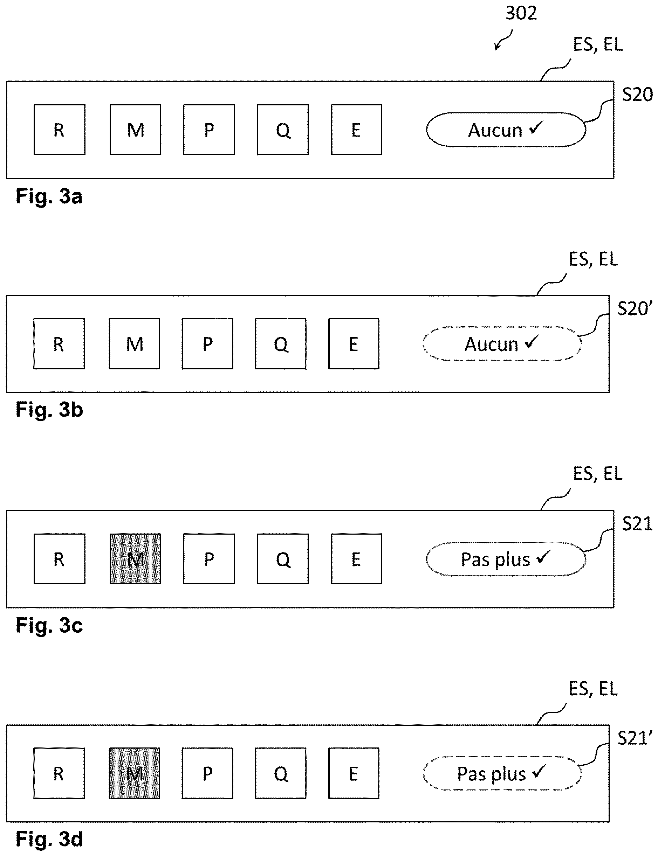

Selon une deuxième étape 302 illustrée par exemple à la figure 3a, le module de commande affiche sur un écran de sélection ES et sur un écran de lecture EL, qui sont deux écrans distincts ou alternativement un seul et même écran, un symbole de test ou une pluralité de symboles de test. Lorsqu'une pluralité de symboles de test est affichée, il s'agit préférentiellement d'une pluralité de symboles de test distincts les uns des autres. Chaque symbole de test peut être :
- une lettre de l'alphabet, ou
- un chiffre, ou
- un dessin, par exemple une voiture, une fleur, une maison, un chat..., ou
- un E de Snellen, également appelé trident de Raskin, ou
- un C de Landolt, également appelé anneau de Landolt.

Le type de symbole de test est avantageusement adapté au profil du sujet examiné : on choisira par exemple un ou plusieurs symboles de test de type dessin si le sujet examiné ne sait pas lire les lettre de l'alphabet (jeune enfant), etc. Le nombre de symboles de test affichés varie préférentiellement de 1 à 7, avec une préférence pour les nombres impairs. Dans l'exemple de la figure 3a, cinq lettres différentes de l'alphabet sont affichées : « R », « M », « P », « Q », « E ».

Lors de la deuxième étape 302, aucun symbole de test n'est sélectionné et tous les symboles de test présentent préférentiellement un même aspect initial. Toujours selon la deuxième étape 302, le module de commande affiche en outre sur l'écran de sélection ES, ou à la fois sur l'écran de sélection ES et sur l'écran de lecture EL, un symbole de validation qui est préférentiellement un premier symbole de validation S20 lorsqu'aucun symbole de test n'est sélectionné, le premier symbole de validation S20 indiquant qu'aucun symbole de test n'est sélectionné. La figure 3a montre un tel exemple de premier symbole de validation S20.

Le procédé 300 comporte ensuite une première décision Q10 selon laquelle le module de commande répond à la question : « le module de commande a-t-il reçu une sélection d'un premier symbole de test ? ».
- Si oui, c'est-à-dire si l'opérateur a sélectionné un premier symbole de test et le module de commande a reçu la sélection dudit premier symbole de test par l'opérateur, le module de commande sélectionne ledit premier symbole de test selon une troisième étape 303. Le symbole de validation est préférentiellement un deuxième symbole de validation S21 lorsqu'au moins un symbole de test est sélectionné donc le module de commande remplace préférentiellement le premier symbole de validation par le deuxième symbole de validation S21 dès lors que le premier symbole de test est sélectionné par l'opérateur. La figure 3c montre un tel deuxième symbole de validation S21. Alternativement, le module de commande peut afficher un même symbole de validation indépendamment de la sélection ou absence de sélection d'un ou plusieurs symboles de test. Le procédé 300 se poursuit ensuite par une deuxième décision Q11
- Sinon, c'est-à-dire si l'opérateur n'a sélectionné aucun symbole de test, le procédé 300 se poursuit par une troisième décision Q12.

Selon la troisième décision Q12, le module de commande répond à la question : « un premier temps prédéterminé s'est-il écoulé depuis la deuxième étape 302 d'affichage ? »
- Si oui, selon une quatrième étape 304, le module de commande modifie au moins une caractéristique d'aspect du symbole de validation, en l'occurrence préférentiellement le premier symbole de validation S20. La figure 3b montre un tel premier symbole de validation modifié S20'.
- Sinon, le procédé 300 se poursuit en bouclant sur la première décision Q10.

Selon la deuxième décision Q11, le module de commande répond à la question : « le premier temps prédéterminé s'est-il écoulé depuis la dernière sélection d'un symbole de test ? ».
- Si oui, selon la quatrième étape 304, le module de commande modifie au moins une caractéristique d'aspect du symbole de validation, en l'occurrence préférentiellement le deuxième symbole de validation S21. La figure 3d montre un tel deuxième symbole de validation modifié S21'.
- Sinon, le procédé 300 se poursuit par une quatrième décision Q13.

Selon la quatrième décision Q13, le module de commande répond à la question : « le module de commande a-t-il reçu une sélection d'un nouveau symbole de test ? ».
- Si oui, c'est-à-dire si l'opérateur a sélectionné un nouveau symbole de test et le module de commande a reçu la sélection dudit nouveau symbole de test par l'opérateur, le procédé 300 reboucle sur la troisième étape 303 selon laquelle le module de commande sélectionne ledit nouveau symbole de test. On entend par « nouveau symbole de test » un symbole de test sélectionné par l'opérateur et/ou le module de commande alors qu'au moins un premier symbole de test est déjà sélectionné.
- Sinon, le procédé 300 reboucle sur la deuxième décision Q11.

Selon la première alternative du procédé 300 qui vient d'être décrite, le module de commande calcule dans la deuxième décision Q11 si le premier temps prédéterminé s'est écoulé depuis la dernière sélection d'un symbole de test ou, si aucun symbole de test n'a été sélectionné, calcule dans la troisième décision Q12 si le premier temps prédéterminé s'est écoulé depuis l'affichage du symbole de test ou de la pluralité de symboles de test. Selon la deuxième alternative illustrée à la figure 1b, le module de commande calcule dans la deuxième décision Q12 si le premier temps prédéterminé s'est écoulé depuis l'affichage du symbole de test ou de la pluralité de symboles de test, indépendamment de la question de la sélection ou absence de sélection d'un ou plusieurs symboles de test.

Le procédé 300 selon la deuxième alternative comporte ainsi la première étape 301 et la deuxième étape 302 telles que précédemment décrites puis la quatrième décision Q13 selon laquelle le module de commande répond à la question : « le module de commande a-t-il reçu une sélection d'un nouveau symbole de test ? ». Si oui, le procédé 300 continue avec la troisième étape 303 précédemment décrite puis avec la troisième décision Q12. Sinon, le procédé 300 se poursuit directement avec la troisième décision Q12. Selon la troisième décision Q12, le module de commande répond à la question : « le premier temps prédéterminé s'est-il écoulé depuis la deuxième étape 302 d'affichage ? ». Si oui, le procédé 300 continue avec la troisième étape 304 précédemment décrite. Sinon, le procédé 300 reboucle sur la quatrième décision Q13.

Selon une variante non illustrée du procédé 300 selon l'une quelconque de ses alternatives, le module de commande lorsqu'il reçoit une sélection d'un symbole de test répond à la question : « le symbole de test sélectionné par l'opérateur est-il actuellement déjà sélectionné par le module de commande ? ». Si oui, le module de commande désélectionne ledit symbole de test. Sinon, si le symbole de test sélectionné par l'opérateur n'était pas déjà sélectionné par le module de commande, le module de commande sélectionne ledit symbole de test.

Dans le cadre de la présente demande, une modification d'au moins une caractéristique d'aspect d'un symbole de test peut notamment être :
- un changement de couleur, et/ou
- un surlignage, et/ou
- l'ajout d'un encadrement ou, si l'encadrement est déjà présent, une modification d'un type de trait de l'encadrement, etc.

Dans le cadre de la présente demande, une modification d'au moins une caractéristique d'aspect du symbole de validation peut notamment être :
- un passage d'un affichage fixe à un affichage clignotant, et/ou
- un changement de dimensions et notamment une augmentation de taille dudit symbole de validation, et/ou
- un changement de couleur, et/ou
- un surlignage, et/ou
- l'ajout d'un encadrement ou, si l'encadrement est déjà présent, une modification d'un type de trait de l'encadrement, etc.

Les figures 3a, 3b, 3c et 3d montrent un exemple d'une succession de différents affichages réalisés par le module de commande.
- Sur la figure 3a, aucun symbole de test n'est sélectionné et le premier symbole de validation S20 correspondant est affiché. Il s'agit par exemple de l'affichage de la deuxième étape 302.
- Sur la figure 3b, le module de commande modifie l'affichage du symbole de validation, en l'occurrence le premier symbole de validation S20 qui devient le premier symbole de validation modifié S20', car le premier temps prédéterminé s'est écoulé, dans la première alternative comme dans la deuxième alternative, depuis l'affichage de la deuxième étape 302 sans qu'aucun symbole de test ne soit sélectionné.
- Sur la figure 3c, l'opérateur sélectionne un premier symbole de test : par exemple la lettre « M ». Le module de commande valide la sélection de l'opérateur et modifie l'aspect du premier symbole de test. Le module de commande affiche préférentiellement le deuxième symbole de validation S21.
- Sur la figure 3d, le module de commande modifie l'affichage du symbole de validation, en l'occurrence le deuxième symbole de validation S21 qui devient le deuxième symbole de validation modifié S21', car le premier temps prédéterminé s'est écoulé, dans la première alternative, depuis la sélection du premier symbole sans qu'aucun nouveau symbole ne soit sélectionné, ou dans la deuxième alternative, depuis l'affichage du ou des symboles de test.

### Premier et deuxième temps prédéterminés

Le module de commande modifie au moins une caractéristique d'aspect du symbole de validation dès qu'un premier temps prédéterminé est écoulé. Cela permet d'inciter l'opérateur à sélectionner ledit symbole de validation afin de mettre fin au procédé 300.

Selon la première alternative du procédé 300, le premier temps prédéterminé est calculé par le module de commande à partir de la dernière sélection d'un symbole de test ou à défaut à partir de l'affichage du symbole de test ou de la pluralité de symboles de test selon la première alternative. Dans ce cas, le premier temps prédéterminé est préférentiellement compris entre deux et cinq secondes.

Selon la deuxième alternative du procédé 300, le premier temps prédéterminé est calculé par le module de commande systématiquement à partir de l'affichage du symbole de test ou de la pluralité de symboles de test, indépendamment de la sélection ou absence de sélection d'un ou plusieurs symboles de test. Dans ce cas, le premier temps prédéterminé est préférentiellement compris entre deux et neuf secondes. Le premier temps prédéterminé est avantageusement choisi en fonction du nombre de symboles de test affichés lors de la deuxième étape 302. Ainsi, le premier temps prédéterminé est préférentiellement compris :
- entre deux et quatre secondes pour un seul symbole de test affiché ;
- entre trois et cinq secondes pour deux ou trois symboles de test affichés ;
- entre cinq et huit secondes pour quatre ou cinq symboles de test affichés ;
- entre six et neuf secondes pour six ou sept symboles de test affichés.

Dans la première alternative comme dans la deuxième alternative, le premier temps prédéterminé est préférentiellement de l'ordre de grandeur de la seconde, c'est-à-dire compris dans l'intervalle [0,1 seconde ; 20 secondes], plus préférentiellement [0,1 seconde; 10 secondes], plus préférentiellement [1 seconde ; 10 secondes].

Dans la première alternative comme dans la deuxième alternative, une augmentation du premier temps prédéterminé peut être prévue selon le profil du sujet examiné et notamment selon son âge, en particulier si le sujet examiné est jeune, par exemple 10 ans ou moins, ou âgé, par exemple 70 ans ou plus. L'augmentation du premier temps prédéterminé peut être indépendante du premier temps prédéterminé, par exemple cinq secondes supplémentaires ajoutées au premier temps prédéterminé quel que soit le premier temps prédéterminé, ou fonction du premier temps prédéterminé, par exemple en doublant le premier temps prédéterminé.

Selon l'une quelconque des alternatives du procédé 300, lorsque le premier temps prédéterminé est écoulé, le module de commande modifie au moins une caractéristique d'aspect du symbole de validation. Selon une variante du procédé 300, lorsqu'un deuxième temps prédéterminé, calculé par le module de commande à partir de ladite modification d'au moins une caractéristique du symbole de validation, est écoulé, le module de commande sélectionne le symbole de validation et met fin automatiquement au procédé 300 selon le premier aspect de l'invention. Le deuxième temps prédéterminé est préférentiellement compris dans l'intervalle [10 secondes; 20 secondes]. Le deuxième temps prédéterminé peut être indépendant du premier temps prédéterminé, par exemple égal à 15 secondes quel que soit le premier temps prédéterminé, ou fonction du premier temps prédéterminé, par exemple compris entre deux et cinq fois le premier temps prédéterminé. Une augmentation du deuxième temps prédéterminé peut également être prévue en fonction du profil du sujet examiné et notamment en fonction de son âge, de même que pour le premier temps prédéterminé. L'augmentation du deuxième temps prédéterminé peut être indépendante du deuxième temps prédéterminé ou fonction du deuxième temps prédéterminé.

### Nombre prédéterminé de symboles de test

Le procédé 300 selon le premier aspect de l'invention prend fin dès qu'un nombre prédéterminé de symboles de test a été sélectionné. Cela permet d'encadrer la mesure d'acuité visuelle en évitant que différents opérateurs successifs choisissent différents critères pour mettre fin au procédé 300. On définit un ratio R du nombre de symboles de test sélectionnés sur le nombre de symboles de test affichés. Le nombre prédéterminé de symboles de test est préférentiellement de :
- un, pour un seul symbole de test affiché - soit un ratio de 100% ;
- un, pour deux symboles de test affichés - soit un ratio de 50% ;
- deux, pour trois symboles de test affichés - soit un ratio de 67% ;
- trois, pour quatre symboles de test affichés - soit un ratio de 75% ;
- trois, pour cinq symboles de test affichés - soit un ratio de 60% ;
- quatre, pour six symboles de test affichés - soit un ratio de 67% ;
- quatre, pour sept symboles de test affichés - soit un ratio de 57%.

### Module de commande selon le deuxième aspect de l'invention

Le module de commande selon le deuxième aspect de l'invention comporte quant à lui des moyens pour afficher simultanément sur l'écran de sélection ES et sur l'écran de lecture EL un symbole de test ou une pluralité de symboles de test, ainsi que des moyens pour afficher un symbole de validation sur l'écran de sélection ES uniquement, ou simultanément sur l'écran de sélection ES et sur l'écran de lecture EL.

Le module de commande selon le deuxième aspect de l'invention comporte également des moyens pour recevoir une sélection d'un symbole de test ou du symbole de validation sur l'écran de sélection ES.

Enfin, le support est préférentiellement une tête de réfracteur automatique et dans ce cas, le module de commande selon le deuxième aspect de l'invention comporte préférentiellement des moyens pour assurer la fonction de console de commande de la tête de réfracteur automatique, en particulier pour agencer un verre optique dans un emplacement de la tête de réfracteur automatique.

Par ailleurs, les moyens d'affichage sur l'écran de sélection ES et l'écran de lecture EL et de réception d'une sélection sur l'écran de sélection ES sont tels que :
- lorsque le module de commande reçoit une sélection d'un symbole de test, le module de commande sélectionne ledit symbole de test en modifiant au moins une caractéristique d'aspect dudit symbole de test de manière qu'il passe d'un aspect initial à un aspect modifié qui le différencie des symboles de test non sélectionnés ;
- le module de commande modifie au moins une caractéristique d'aspect du symbole de validation, de manière qu'il passe d'un aspect initial à un aspect modifié, lorsqu'un premier temps prédéterminé s'est écoulé depuis la dernière sélection d'un symbole de test ou depuis l'affichage du symbole de test ou de la pluralité de symboles de test ;
- le module de commande met fin au procédé de test visuel dès qu'un nombre prédéterminé de symboles de test a été sélectionné.

D'une manière générale, le module de commande comporte avantageusement des moyens pour mettre en œuvre tout ou partie des étapes optionnelles du procédé 300 selon le premier aspect de l'invention.

### Ecran de sélection et écran de lecture

L'opérateur interagit avec l'écran de sélection ES pour sélectionner un symbole de test ou un symbole de validation. Le sujet examiné interagit quant à lui avec l'écran de lecture pour identifier, à travers le support, le ou les symboles de test affichés.

L'écran de sélection ES et l'écran de lecture EL peuvent être deux écrans distincts ou alternativement, un seul et même écran remplissant à la fois les fonctions « sélection » et « lecture ». L'affichage du symbole de test ou de la pluralité de symboles de test, d'aspect modifié en cas de sélection, est identique sur l'écran de sélection ES et sur l'écran de lecture EL de manière que l'opérateur et le sujet examiné accèdent à la même information. L'affichage du symbole de sélection est réalisé au moins sur l'écran de sélection ES et préférentiellement à la fois sur l'écran de sélection ES et sur l'écran de lecture EL de manière que l'opérateur et le sujet examiné accèdent à la même information.

### Opérateur et sujet examiné

On définit l'opérateur comme étant celui qui interagit avec l'écran de sélection ES et donc avec le module de commande. L'opérateur est typiquement un praticien, tel qu'un ophtalmologue, un opticien ou même, dans le cadre de la présente invention, un assistant sans compétences particulières en physique de la réfraction et physiologie du système visuel. On définit le sujet examiné comme étant celui qui regarde l'écran de lecture à travers le support. Le sujet examiné est typiquement un patient ou client du praticien.

Le sujet examiné peut lui-même être opérateur. Le sujet examiné peut être l'unique opérateur en l'absence de tout praticien. Alternativement, deux opérateurs distincts, par exemple le sujet examiné et un praticien, peuvent se relayer. Lorsque le sujet examiné est opérateur, soit l'écran de lecture forme un seul et même écran avec l'écran de sélection, soit l'écran de lecture est un deuxième écran de sélection.

### Système selon le troisième aspect de l'invention

Le système selon le troisième aspect de l'invention comporte le module de commande selon le deuxième aspect de l'invention ainsi que l'écran de sélection ES et l'écran de lecture EL.

L'écran de sélection ES peut être intégré avec le module de commande. C'est par exemple le cas d'un module de commande sous forme de tablette tactile ou d'ordinateur portable. Alternativement, l'écran de sélection ES peut être distinct du module de commande. C'est par exemple le cas d'un module de commande connecté avec un écran distant de type écran d'ordinateur, écran de télévision ou écran de projection.

L'écran de sélection ES et l'écran de lecture EL peuvent être un seul et même écran ou deux écrans distincts.

### Moyens d'interaction

Chaque étape de sélection d'un symbole de test ou de validation est réalisée par l'opérateur réalisant une action de sélection en interaction avec l'écran de sélection ES et donc avec le module de commande. Chaque action de sélection peut être réalisée de différentes manières, dont plusieurs exemples non limitatifs sont listés ci-après.

L'action de sélection est préférentiellement réalisée au moyen d'un écran de sélection ES tactile : l'opérateur touche une zone de l'écran sur laquelle se trouve une ligne ou un symbole de validation afin de sélectionner ladite ligne ou ledit symbole de validation.

Alternativement, l'action de sélection peut être réalisée au moyen d'une interface qui est par exemple
- de type souris : l'opérateur positionne le curseur de la souris sur la zone de l'écran sur laquelle se trouve le symbole de test ou de validation et clique sur cette zone afin de sélectionner ledit symbole de test ou de validation ; ou
- de type clavier : chaque symbole de test ou de validation est identifié par une touche ou combinaison de touches qui lui est propre, par exemple la touche « R » pour le premier symbole de test qui est la lettre « R », la touche « M » pour le deuxième symbole de test qui est la lettre « M », etc. Une seule et même touche ou combinaison de touches, par exemple la touche « entrée », identifie avantageusement le symbole de validation. L'opérateur appuie sur une touche ou combinaison de touches afin de sélectionner le symbole de test ou de validation correspondant ;
- de type manette directionnelle ou joystick : chaque symbole de test est identifié par une direction qui lui est propre et l'opérateur oriente la manette dans une direction donnée afin de sélectionner le symbole de test correspondant. Cette alternative est particulièrement adaptée aux symboles de test caractérisables par une direction, comme les symboles de test de type « E de Snellen » ou « C de Landolt » : chaque « E » de Snellen est caractérisé par la direction de sa barre centrale, chaque « C » de Landolt est caractérisé par la direction de son ouverture. La manette directionnelle ou joystick peut avantageusement avoir la forme du type symbole de test affiché, notamment la forme d'un « E » de Snellen ou d'un « C » de Landolt, afin de faciliter son orientation par l'opérateur. Le symbole de validation est quant à lui préférentiellement identifié par un bouton qui lui est propre et l'opérateur appuie sur le bouton afin de sélectionner le symbole de validation.

Selon une autre alternative, l'action de sélection est réalisée au moyen d'une commande vocale, via un équipement de type microphone et un module de traitement du signal adapté dans le module de commande : chaque symbole de test ou de validation est identifié par un mot ou groupe de mots qui lui est propre, par exemple prononcer la lettre « R » pour le premier symbole de test qui est la lettre R, prononcer la lettre « M » pour le deuxième symbole de test qui est la lettre « M », etc. Un seul et même mot ou groupe de mot, par exemple le mot « valider » ou « aucun », ou « pas d'autre », etc., identifie avantageusement le symbole de validation. L'opérateur prononce un mot ou groupe de mots afin de sélectionner le symbole de test ou de validation correspondant. Cette alternative est particulièrement adaptée au cas où le sujet examiné est opérateur.

Selon encore une autre alternative, la sélection est réalisée au moyen d'une commande gestuelle, via un équipement de type caméra et un module de traitement du signal adapté dans le module de commande. Chaque symbole de test ou de validation est identifié par une position ou mouvement de la main qui lui est propre, par exemple le poing fermé pour sélectionner le symbole de validation. L'opérateur positionne sa main ou réalise un mouvement afin de sélectionner le symbole de test ou de validation correspondant. Cette alternative est particulièrement adaptée aux symboles de test, comme les symboles de test de type « E de Snellen » ou « C de Landolt », caractérisables par une direction : par exemple la direction dans laquelle pointe la barre centrale de chaque « E » de Snellen ou la direction dans laquelle s'ouvre chaque « C » de Landolt. L'opérateur utilise par exemple son pouce levé qu'il oriente dans une direction donnée afin de valider le symbole de test correspondant.

Les différentes actions de sélection du procédé 300 selon le premier aspect de l'invention peuvent être toutes réalisées d'une seule et même manière, par exemple au moyen d'un écran de sélection tactile. Alternativement, une combinaison de plusieurs techniques différentes peut être utilisée pour réaliser les différentes actions de sélection du procédé 300, par exemple les actions de sélection d'un symbole de test via un écran de sélection tactile ou via une commande vocale, et les actions de sélection d'un symbole de validation via une commande gestuelle ou un périphérique, etc.

## Revendications

1. Procédé (300) de test visuel comportant une étape d'agencement d'un verre optique dans un emplacement d'un support et les étapes suivantes selon lesquelles un module de commande :
- affiche (302) un symbole de test ou une pluralité de symboles de test sur un écran de lecture (EL) et sur un écran de sélection (ES) et affiche en outre un symbole de validation au moins sur l'écran de sélection (ES) ;
- reçoit une sélection d'un symbole de test ou du symbole de validation sur l'écran de sélection (ES) ;
le procédé **caractérisé en ce que** :
- lorsque (Q10, Q13) le module de commande reçoit une sélection d'un symbole de test, le module de commande sélectionne (303) ledit symbole de test en modifiant au moins une caractéristique d'aspect dudit symbole de test de manière qu'il passe d'un aspect initial à un aspect modifié qui le différencie des symboles de test non sélectionnés ;
- lorsque (Q11, Q12) un premier temps prédéterminé s'est écoulé depuis la dernière sélection d'un symbole de test ou depuis l'affichage du symbole de test ou de la pluralité de symboles de test, le module de commande modifie (304) au moins une caractéristique d'aspect du symbole de validation ;
- le module de commande met fin au procédé de test visuel dès qu'un nombre prédéterminé de symboles de test a été sélectionné.

2. Procédé (300) selon la revendication précédente, **caractérisé en ce que** lorsque (Q12) le premier temps prédéterminé s'est écoulé depuis l'affichage du symbole de test ou de la pluralité de symboles de test, indépendamment de la sélection ou absence de sélection d'un ou plusieurs symboles de test, le module de commande modifie (304) au moins une caractéristique d'aspect du symbole de validation.

3. Procédé (300) selon l'une quelconque des revendications précédentes, **caractérisé en ce que** lorsque le module de commande reçoit une sélection d'un symbole de test qui est déjà sélectionné, le module de commande désélectionne ledit symbole de test en lui redonnant son aspect initial.

4. Procédé (300) selon l'une quelconque des revendications précédentes, **caractérisé en ce que** le module de commande met automatiquement fin au procédé de test visuel lorsqu'un deuxième temps prédéterminé s'est écoulé depuis la modification (304) d'au moins une caractéristique d'aspect du symbole de validation par le module de commande.

5. Procédé (300) selon l'une quelconque des revendications précédentes, **caractérisé en ce que** le symbole de validation est un premier symbole de validation tant qu'aucun symbole de test n'a été sélectionné, ou un deuxième symbole de validation, distinct du premier symbole de validation, dès qu'au moins un symbole de test est sélectionné.

6. Procédé (300) selon l'une quelconque des revendications précédentes, **caractérisé en ce que** chaque symbole de test est :
- une lettre, ou
- un chiffre, ou
- un dessin, ou
- un E de Snellen, ou
- un C de Landolt.

7. Module de commande pour la mise en œuvre du procédé (300) de test visuel selon l'une quelconque des revendications précédentes, le module de commande ayant :
- des moyens pour afficher un symbole de test ou une pluralité de symboles de test sur un écran de lecture (EL) et sur un écran de sélection (ES) ;
- des moyens pour afficher un symbole de validation au moins sur l'écran de sélection (ES) ;
- des moyens pour recevoir une sélection d'un symbole de test ou du symbole de validation sur l'écran de sélection ;
les moyens d'affichage sur l'écran de sélection (ES) et l'écran de lecture (EL) et de réception d'une sélection sur l'écran de sélection (ES) **caractérisé en ce que** :
- lorsque le module de commande reçoit une sélection d'un symbole de test, le module de commande sélectionne ledit symbole de test en modifiant au moins une caractéristique d'aspect dudit symbole de test de manière qu'il passe d'un aspect initial à un aspect modifié qui le différencie des symboles de test non sélectionnés ;
- le module de commande modifie au moins une caractéristique d'aspect du symbole de validation, de manière qu'il passe d'un aspect initial à un aspect modifié, lorsque le premier temps prédéterminé s'est écoulé depuis la dernière sélection d'un symbole de test ou depuis l'affichage du symbole de test ou de la pluralité de symboles de test ;
- le module de commande met fin au procédé de test visuel dès qu'un nombre prédéterminé de symboles de test a été sélectionné.

8. Système pour la mise en œuvre d'un procédé (300) de test visuel selon l'une quelconque des revendications 1 à 6, comportant le module de commande selon la revendication 7 ainsi que l'écran de sélection (ES) et l'écran de lecture (EL).

9. Système selon la revendication précédente **caractérisé en ce que** l'écran de sélection (ES) et l'écran de lecture (EL) sont deux écrans distincts.

10. Système selon la revendication 8 **caractérisé en ce que** l'écran de sélection (ES) et l'écran de lecture (EL) sont un seul et même écran.

## Patentansprüche

1. Visuelles Testverfahren (300), umfassend einen Anordnungsschritt eines optischen Glases an einer Stelle eines Trägers und die folgenden Schritte, gemäß denen ein Steuermodul:
- ein Testsymbol oder eine Vielzahl von Testsymbolen auf einem Lesebildschirm (EL) und auf einem Auswahlbildschirm (ES) anzeigt (302) und darüber hinaus ein Bestätigungssymbol wenigstens auf dem Auswahlbildschirm (ES) anzeigt,
- eine Auswahl eines Testsymbols oder des Bestätigungssymbols auf dem Auswahlbildschirm (ES) empfängt;
das Verfahren **dadurch gekennzeichnet ist, dass**:
- wenn (Q10, Q13) das Steuermodul eine Auswahl eines Testsymbols empfängt, das Steuermodul das genannte Testsymbol auswählt (303), indem es wenigstens ein Erscheinungsbildmerkmal des genannten Testsymbols derart abändert, dass es von einem ursprünglichen Erscheinungsbild zu einem abgeänderten Erscheinungsbild übergeht, das es von nicht auswählten Testsymbolen unterscheidet;
- wenn (Q11, Q12) eine erste vorbestimmte Zeit seit der letzten Auswahl eines Testsymbols oder seit der Anzeige des Testsymbols oder der Vielzahl von Testsymbolen verstrichen ist, ändert (304) das Steuermodul wenigstens ein Erscheinungsbildmerkmal des Bestätigungssymbols ab;
- das Steuermodul das visuelle Testverfahren beendet, sobald eine vorbestimmte Anzahl von Testsymbolen ausgewählt wurde.

2. Verfahren (300) gemäß dem voranstehenden Anspruch, **dadurch gekennzeichnet, dass**, wenn (Q12) die erste vorbestimmte Zeit seit der Anzeige des Testsymbols oder der Vielzahl von Testsymbolen unabhängig von der Auswahl oder dem Fehlen der Auswahl eines oder mehrerer Testsymbol(e) verstrichen ist, das Steuermodul wenigstens ein Erscheinungsbildmerkmal des Bestätigungssymbols abändert (304).

3. Verfahren (300) gemäß irgendeinem der voranstehenden Ansprüche, **dadurch gekennzeichnet, dass**, wenn das Steuermodul eine Auswahl eines Testsymbols empfängt, das bereits ausgewählt ist, das Steuermodul das genannte Testsymbol abwählt, indem es ihm sein ursprüngliches Erscheinungsbild zurückgibt.

4. Verfahren (300) gemäß irgendeinem der voranstehenden Ansprüche, **dadurch gekennzeichnet, dass** das Steuermodul das visuelle Testverfahren automatisch beendet, wenn eine zweite vorbestimmte Zeit seit der Abänderung (304) wenigstens eines Erscheinungsbildmerkmals des Bestätigungssymbols durch das Steuermodul abgelaufen ist.

5. Verfahren (300) gemäß irgendeinem der voranstehenden Ansprüche, **dadurch gekennzeichnet, dass** das Bestätigungssymbol ein erstes Bestätigungssymbol, solange kein Testsymbol ausgewählt wurde, oder ein zweites Bestätigungssymbol, das von dem ersten Bestätigungssymbol unterschiedlich ist, sobald wenigstens ein Testsymbol ausgewählt wird, ist.

6. Verfahren (300) gemäß irgendeinem der voranstehenden Ansprüche, **dadurch gekennzeichnet, dass** jedes Testsymbol ist:
- ein Buchstabe oder
- eine Zahl oder
- eine Zeichnung oder
- ein Snellen-E oder
- ein Landolt-C.

7. Steuermodul für die Umsetzung des visuellen Testverfahrens (300) gemäß irgendeinem der voranstehenden Ansprüche, wobei das Steuermodul aufweist:
- Mittel zum Anzeigen eines visuellen Testsymbols oder einer Vielzahl von Testsymbolen auf einem Lesebildschirm (EL) und auf einem Auswahlbildschirm (ES);
- Mittel zum Anzeigen eines Bestätigungssymbols wenigstens auf dem Auswahlbildschirm (ES);
- Mittel zum Empfangen einer Auswahl eines Testsymbols oder des Bestätigungssymbols auf dem Auswahlbildschirm;
wobei die Mittel zum Anzeigen auf dem Auswahlbildschirm (ES) und dem Lesebildschirm (EL) und zum Empfangen einer Auswahl auf dem Auswahlbildschirm (ES) **dadurch gekennzeichnet sind, dass**:
- wenn das Steuermodul eine Auswahl eines Testsymbols empfängt, das Steuermodul das genannte Testsymbol auswählt, indem es wenigstens ein Erscheinungsbildmerkmal des genannten Testsymbols derart abändert, dass es von einem ursprünglichen Erscheinungsbild zu einem abgeänderten Erscheinungsbild übergeht, das es von den nicht ausgewählten Testsymbolen unterscheidet;
- das Steuermodul wenigstens ein Erscheinungsbildmerkmal des Bestätigungssymbols derart abändert, dass es von einem ursprünglichen Erscheinungsbild zu einem abgeänderten Erscheinungsbild übergeht, wenn die erste vorbestimmte Zeit seit der letzten Auswahl eines Testsymbols oder seit der Anzeige des Testsymbols oder der Vielzahl von Testsymbolen verstrichen ist;
- das Steuermodul das visuelle Testverfahren beendet, sobald eine vorbestimmte Anzahl von Testsymbolen ausgewählt wurde.

8. System für die Umsetzung eines visuellen Testverfahrens (300) gemäß irgendeinem der Ansprüche 1 bis 6, umfassend das Steuermodul gemäß Anspruch 7, sowie den Auswahlbildschirm (ES) und den Lesebildschirm (EL).

9. System gemäß dem voranstehenden Anspruch, **dadurch gekennzeichnet, dass** der Auswahlbildschirm (ES) und der Lesebildschirm (EL) zwei unterschiedliche Bildschirme sind.

10. System gemäß Anspruch 8, **dadurch gekennzeichnet, dass** der Auswahlbildschirm (ES) und der Lesebildschirm (EL) ein und derselbe Bildschirm sind.

## Claims

1. Visual test method (300) comprising a step of arranging an optical lens in a slot of a support and the following steps according to which a control module:
- displays (302) one test symbol or a plurality of test symbols on a reading screen (EL) and on a selection screen (ES) and further displays one validation symbol at least on the selection screen (ES);
- receives a selection of one test symbol or of the validation symbol on the selection screen (ES);
the method **characterized in that**:
- when (Q10, Q13) the control module receives a selection of one test symbol, the control module selects (303) said test symbol by modifying at least one appearance characteristic of said test symbol in such a way that it changes from an initial appearance to a modified appearance which differentiates it from non-selected test symbols;
- when (Q11, Q12) a first predetermined time has elapsed since the last selection of one test symbol or since the display of the test symbol or the plurality of test symbols, the control module modifies (304) at least one appearance characteristic of the validation symbol;
- the control module ends the visual test method as soon as a predetermined number of test symbols has been selected.

2. Method (300) according to the preceding claim, **characterised in that** when (Q12) the first predetermined time has elapsed since the display of the test symbol or the plurality of test symbols, independently of the selection or absence of selection of one or more test symbols, the control module modifies (304) at least one appearance characteristic of the validation symbol.

3. Method (300) according to any one of the preceding claims, **characterised in that** when the control module receives a selection of one test symbol which is already selected, the control module deselects said test symbol, giving it back its initial appearance.

4. Method (300) according to any one of the preceding claims, **characterised in that** the control module automatically ends the visual test method when a second predetermined time has elapsed since the modification (304) of at least one appearance characteristic of the validation symbol by the control module.

5. Method (300) according to any one of the preceding claims, **characterised in that** the validation symbol is a first validation symbol as long as no test symbol has been selected, or a second validation symbol, distinct from the first validation symbol, as soon as at least one test symbol is selected.

6. Method (300) according to any one of the preceding claims, **characterised in that** each test symbol is:
- a letter, or
- a figure, or
- a drawing, or
- a Snellen E, or
- a Landolt C.

7. Control module for the implementation of the visual test method (300) according to any one of the preceding claims, the control module having:
- means for displaying one test symbol or a plurality of test symbols on a reading screen (EL) and on a selection screen (ES);
- means for displaying one validation symbol at least on the selection screen (ES);
- means for receiving a selection of one test symbol or of the validation symbol on the selection screen;
the display means on the selection screen (ES) and the reading screen (EL) and the means for receiving a selection on the selection screen (ES) being **characterized in that**:
- when the control module receives a selection of one test symbol, the control module selects said test symbol by modifying at least one appearance characteristic of said test symbol in such a way that it changes from an initial appearance to a modified appearance which differentiates it from non-selected test symbols;
- the control module modifies at least one appearance characteristic of the validation symbol, in such a way that it changes from an initial appearance to a modified appearance, when the first predetermined time has elapsed since the last selection of one test symbol or since the display of the test symbol or the plurality of test symbols;
- the control module ends the visual test method as soon as a predetermined number of test symbols has been selected.

8. System for the implementation of a visual test method (300) according to any one of claims 1 to 6, comprising the control module according to claim 7 as well as the selection screen (ES) and the reading screen (EL).

9. System according to the preceding claim **characterised in that** the selection screen (ES) and the reading screen (EL) are two distinct screens.

10. System according to claim 8 **characterised in that** the selection screen (ES) and the reading screen (EL) are a single and same screen.
